# EUROPEAN PATENT SPECIFICATION

(11) **EP 1 544 311 B1**
(45) Date of publication and mention of the grant of the patent: **18.11.2009**
(21) Application number: 03293185.9
(22) Date of filing: 16.12.2003
(51) Int. Cl.: C12Q 1/70

(54) **Oligonucleotides for the detection of hepatitis B virus**
Oligonukleotide zur Detektion von Hepatitis B Viren
Oligonucléotides pour la détection du virus de l'hépatite B

(43) Date of publication of application: 22.06.2005
(73) Proprietor: Bio-Rad Pasteur, 92430 Marnes-la-Coquette (FR)
(72) Inventor: Biron, Marie-Philippe, 92600 Asnières sur Seine (FR)
(74) Representative: Bernasconi, Jean Raymond

(56) References cited:
- WO-A-93/13120
- WO-A-95/17414
- WO-A-03/093797
- US-A- 5 877 162
- US-A1- 2003 157 478
- DATABASE EMBL [Online] EBI; 22 February 2001 (2001-02-22), MASAKAZU M, SHUICHI K, KAZUMASA H: "Primer for detecting hepatitis B virus and method for detecting hepatitis B virus therewith" XP002282772 Database accession no. E28674 & PATENT ABSTRACTS OF JAPAN vol. 1996, no. 04, 30 April 1996 (1996-04-30) & JP 7 327686 A (IMMUNO JAPAN:KK), 19 December 1995 (1995-12-19) & JP 07 327686 A (IMMUNO JAPAN:KK) 19 December 1995 (1995-12-19)
- DATABASE GENESEQ [Online] EBI; PCR primer Hepatitis B virus X-region 23 May 1996 (1996-05-23), UCHIDA T, SHIKATA T: XP002282773 Database accession no. AAT08233 & DATABASE EPODOC EUROPEAN PATENT OFFICE, THE HAGUE, NL; 19 October 1995 (1995-10-19), & WO 95/27788 A (DAINABOT CO LTD ; SHIKATA TOSHIO (JP); UCHIDA TOSHIKAZU (JP)) 19 October 1995 (1995-10-19)
- DATABASE EMBL [Online] EBI; 24 January 1995 (1995-01-24), XP002282774 Database accession no. I00797

## Description

The invention relates to oligonucleotide primers and probes for rapid and sensitive detection of hepatitis B virus by amplification, and to methods using the same.

Hepatitis B virus (HBV) is a member of the hepadnavirus family. These are double-stranded DNA viruses which replicate, unusually, by reverse transcription. A number of variants of this virus have been described. HBV is a major causative agent of chronic hepatitis and has been implicated in liver cirrhosis and hepatocellular carcinoma. Hepatitis B virus is endemic in the human population and hyperendemic in many parts of the world. It is estimated that more than one third of the world's population has been infected with the hepatitis B virus. About 5% of the population are chronic carriers of HBV, and nearly 25% of all carriers develop serious liver diseases such as chronic hepatitis, cirrhosis, and primary hepatocellular carcinoma. HBV infection causes more than one million deaths every year:

Antibody and cell-mediated immune responses to various types of antigens are induced during the infection. The immune response to infection with hepatitis B virus is directed toward at least three antigens: hepatitis B surface antigen, the core antigen (HBcAg), and the e antigen (HBeAg). The surface antigen appears in the sera of most patients during the late stage of the incubation period, i.e. 2-8 weeks post-infection, and persists during the acute illness and sharply decreases when antibody to the surface antigen becomes detectable. IgM to the core antigen is found in the serum 2-10 weeks after the surface antigen appears. It correlates with the amount and duration of virus replication. As to Hepatitis B e antigen, it is secreted by infected hepatocytes but eventually, in most individuals, seroconversion from HBeAg to anti-HBe is observed, associated with the immune clearance of infected hepatocytes.

Immunoassays have been developed to detect and measure HBV antigens and antibodies.

However, such immunoassays may generate false negative results. Indeed, where an immunological method is carried out within the seroconversion phase of the subject, HBV infection may remain undetected as none circulating antigen or antibodies would be found. Accordingly, diagnostic methods relying on the detection of HBV nucleic acid provide a more accurate method allowing for an earlier detection of the virus

US 2003/0157478 discloses a method and kit for diagnosing HBV by PCR and hybridisation to a detection probe.

Several diagnostic kits have been marketed in this field, for example, Amplicor HBV Monitor and Cobas Amplicor HBV Monitor (Roche Molecular Systems), Versant HBV DNA (bDNA, Bayer Diagnostics) and Digene Hybrid-Capture 2 HBV DNA test (Digene Corporation). However, these tests have generally a limited range of quantification with no more than 4 log: 10³ to 4 10⁶ copies/ml for Amplicor HBV Roche (2 10² to 2 10⁵ copies/ml for Cobas HBV Roche), 7 10⁵ to 5 10⁹ copies/ml for Versant HBV Bayer and 7 10⁵ to 5.6 10⁹ copies/ml for Digene HBV DNA (4.7 10³ to 5.7 10⁷ copies/ml for Ultrasensitive Digene HBV DNA). Thus, the main disadvantage of all of these tests resides in the fact that none of them makes it possible to clearly and simply quantify HBV from very low to very high concentrations. Therefore, it is indeed often necessary to combine at least two tests in order to obtain an accurate result, particularly in the case of low or high viral concentration level.

Thus, there is a need for accurate quantification of HBV in a large range, whatever the concentration of the virus in a sample and without the need to perform any additional test.

In addition there is still a need for a very sensitive quantitative test that specifically detects all HBV genotypes known in the art.

In this context, the inventors have designed primers and probe that provide particularly rapid and sensitive means for detecting HBV nucleic acid from genotypes A to G with a very wide range of quantification.

### Definitions

In accordance with the present invention there may be employed any conventional molecular biology, microbiology, and recombinant DNA techniques within the skill of the art. Such techniques are explained fully in the literature. See, e.g., Sambrook et al., 1989 ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, 1984; F.M. Ausubel et al. (eds.), 1994.

A *"nucleic acid molecule"* refers to any nucleic acid : it may be synthetic or not, recombinant or naturally occurring, linear or circular. It may be either in single stranded or in double stranded form. These nucleic acid molecules include genomic DNA, cDNA or RNA. Unless otherwise specified, sequences are described according to the normal convention of giving only the sequence in the 5' to 3' direction along the non-transcribed strand of DNA (i.e., the strand having a sequence homologous to the mRNA).

As used herein, the term *"oligonucleotide"* refers to a nucleic acid sequence, which can be used as primer in an amplification procedure or as probe in a method of detection. In the context of the invention, these oligonucleotides consists of at least 10, preferably at least 15, and more preferably at least 20 nucleotides, preferably no more than 100 nucleotides, more preferably no more than 40 nucleotides that are hybridizable to a genomic DNA molecule, a cDNA molecule, or a mRNA molecule encoding a gene, mRNA, cDNA, or other nucleic acid of interest.

A nucleic acid molecule is *"hybridizable"* to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989). The conditions of temperature and ionic strength determine the *"stringency"* of the hybridization. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989, II.7-11.8). A minimum length for a hybridizable nucleic acid is at least about 10 nucleotides, preferably at least about 15 nucleotides, and more preferably the length is at least about 20 nucleotides.

PCR is performed under high stringency conditions, by using an annealing temperature of at least 50°C in a high ionic strength buffer. According to a preferred embodiment of the invention, the temperature chosen for the annealing step ranges from 55°C to 60°C and the ionic strength is obtained by using a combination of KCI and MgCl2 which promotes a high ratio of specific to non specific oligonucleotide binding during the annealing step of each PCR cycle. This enables for stringent primer annealing conditions, leading to increased PCR specificity. For instance, a combination of KCI 50 mM and MgCl2 6 mM may be appropriate to confer high specificity. However, other ionic strength conditions may be used and can be readily determined by the one skilled in the art.

*"Amplification"* of DNA, as used herein, denotes the increase in the concentration of a particular DNA sequence within a mixture of DNA sequences. The amplification step may be carried out by any method using conventional methods of enzymatic amplification of DNA or RNA, such as in particular the TAS (Transcription-based Amplification System) technique proposed by Kwoh et al. (1989), the 3SR (Self-Sustained Sequence Replication) technique described by Fahy et al. (1991), the NASBA (Nucleic Acid Sequence-Based Amplification) technique described in patent EP 329 822, or alternatively the SDA (Strand Displacement Amplification) technique described by Walker et al. (1992), or the Ligase Chaine Reaction (LCR) technique described in European patent EP 0 320 308, or the Transcription Mediated Amplification (TMA) described in US 5,399,491, or advantageously the PCR technique as described by Saiki et al. (1988) and in European patents EP 0 200 362 and EP 0 201 184, or alternatively the techniques derived from the latter and any other method desired for amplifying nucleic sequences in vitro.

The use of polymerase chain reaction (PCR) is more particularly contemplated in the context of the invention.

As used herein, the terms "*primer*" and "*probe*" refer to the function of the oligonucleotide. A primer is an oligonucleotide used for amplifying a target sequence typically by extension of the oligonucleotide after hybridization to the target sequence or by ligation of multiple oligonucleotides which are adjacent when hybridized to the target sequence. A probe oligonucleotide is used to capture or detect a target sequence to which it hybridizes. However the same oligonucleotide probe may also function as a primer. It will therefore be appreciated that any of the sequences disclosed herein for amplification, detection or quantitation of HBV may be used either as hybridization probes or as amplification primers for detection or amplification.

The term *"hepatitis B virus"* or *"HBV"* denotes the type species of the genus Orthohepadnavirus, family Hepadnaviridae. HBV nucleic acid is a partially double stranded and single stranded circular DNA. The total genome length is about 3020-3320 nucleotides (nt) (for the full length strand), or 1700-2800 nt (for the short length strand). As used herein, HBV is meant for any subtype, strain, or variant, e.g. subtype *ad, adr, adw, adyw, ar, ayw.* In the context of the invention, nucleotide positions are indicated by reference to the HBV genome sequence, subtype *adw,* deposited at GenBank under accession number X98077 (SEQ ID NO:1).

The term *"biological sample"* refers to any body fluid, such as urine, sang, serum, plasma, LCR or tissues like liver biopsy...

### - Probes and primers of the invention

The inventors have demonstrated that a particularly rapid and sensitive detection of HBV infection can be achieved by amplifying a HBV nucleic acid using the specific primers and probe as defined below.

These primers and probes have been designed to hybridize to an overlapping region of HBV genome, *i.e.* a region that includes the end of the HBV DNA polymerase encoding region (nucleotides (nt) 2307-3215/1-1623 of HBV circularised genome) and the beginning of the sequence encoding the HBV X protein (nt 1374-1838). These primers and probe allow the amplification and the detection of a 162 nucleotide fragment of the HBV genome (region spanning from nt 1440 to 1602 of SEQ ID NO:1).

The detection tests developed herein prove to be of high interest for viral load testing of HBV patients or for the safety screening of blood products

The invention thus provides an oligonucleotide that consists of sequence 5' GCTGAATCCCGCGGACGA 3', (SEQ ID NO:2) or its complementary sequence. This oligonucleotide is particularly useful as a sense primer for amplifying a HBV nucleic acid.

The invention also provides an oligonucleotide that consists of a sequence selected from the group consisting of the sequence 5' GTGCAGAGGTGAAGCGAAGTG 3' (SEQ ID NO:3), the sequence 5' GTTCACGGTGGTCGCCATG 3' (SEQ ID NO:4), the sequence 5' CGTTCACGGTGGTCGCCATGC 3' (SEQ ID NO:6), and the sequence 5' CGTTCACGGTGGTCTCCATGC 3' (SEQ ID NO:7), as well- as their complementary sequence. These oligonucleotides are particularly useful as an antisense primer for amplifying a HBV nucleic acid.

Furthermore, the invention also provides an oligonucleotide, useful as a probe, which consists of a HBV binding sequence consisting of an antisense sequence selected from the group consisting of the sequence 5' GGAGTCCGCGTAAAGAGAGGTG 3' (SEQ ID NO:8), the sequence 5' GGAGACCGCGTAAAGAGAGGTG 3' (SEQ ID NO:9), the sequence 5' GGAGTCTGCGTAAAGAGAGGTG 3' (SEQ ID NO:10), and the sequence 5' GGAGACTGCGTAAAGAGAGGTG 3' (SEQ ID NO:11), as well as their complementary sequence.

The oligonucleotides of the invention may possibly comprise additional sequences linked to the 5' and/or 3' terminus of above-designed sequences, and which are foreign to the desired sequence, such as a labelling molecule. Said oligonucleotides are nevertheless capable of hybridizing under high stringency conditions with the complementary nucleic sequences present in HBV, and said additional sequences are unable to hybridize with HBV nucleic acid under standard hybridization conditions.

These additional sequences may serve as a spacer, linker, or sequence for labelling or binding of an enzyme, etc.

The oligonucleotide of the invention especially the probe that consists of a sequence selected from the group consisting of SEQ ID NO:8, SEQ ID NO:9, SEQ ID NO:10, SEQ ID NO:11, and their complementary sequence, may be detectably labelled.

Standard labelling agents (*e.g.* enzyme, radioactive, or fluorescent moieties) may be used for that purpose.

Labelling of the probe is particularly advantageous to facilitate the detection of the amplified nucleic acid, during a "real-time" amplification/detection reaction, i.e. during a PCR process wherein the target sequence is detected and/or quantified while the amplification reaction is occurring.

Such detection may be achieved for instance using the nucleic acid Molecular Beacon technology (Tyagi and Kramer, 1996; Cayouette et al., 1999). According to the Molecular Beacon technology, one of either a fluorophore or quencher moiety is attached to each termini of the probing sequence. In the absence of the target nucleic acid, the arm sequences anneal to each other to thereby form a loop and hairpin stem structure which brings the fluorophore and quencher together. When contacted with target nucleic acid, the complementary probing sequence and target sequence will hybridize. Because the hairpin stem cannot coexist with the rigid double helix that is formed upon hybridization, the resulting conformational change forces the arm sequences apart and causes the fluorophore and quencher to be separated. When the fluorophore and quencher are separated, the fluorescent signal is detectable.

All dyes and quenchers known in the art can be used. According the invention, the dye may be preferably selected from the group consisting of Fam, Tet, Hex, Tamra, Texas Red and Cy5, and the quencher may be preferably selected from the group consisting of Dabcyl, Eclipse Dark Quencher, and Black Hole Quenchers. Such molecules are readily available from Eurogentec, Biosearch Technology, Proligo....

The invention thus also provides an oligonucleotide that consists of a sequence selected from the group consisting of SEQ ID NO:8, SEQ ID NO:9, SEQ ID NO:10, SEQ ID NO:11, and their complementary sequence and carries a fluorophore moiety at one terminus, and a quencher moiety at the other terminus. Spacer sequences are preferably introduced to link said moieties to one of the sequences SEQ ID NO:8, SEQ ID NO:9. SEQ ID NO:10, SEQ ID NO:11, or their complementary sequence, so as to provide an extended probe.

Oligonucleotides consisting of the sequences 5' CGGCAGGAGTCCGCGTAAAGAGAGGTGTGCCG 3' (SEQ ID NO:12), 5' CGGCAGGAGACCGCGTAAAGAGAGGTGTGCCG 3' (SEQ ID NO:13), 5' CGGCAGGAGTCTGCGTAAAGAGAGGTGTGCCG3' (SEQ ID NO:14), and 5' CGGCAGGAGACTGCGTAAAGAGAGGTGTGCCG 3' (SEQ ID NO:15) are an examples of such extended probe, and are also part of the present invention. The specific pair of spacer sequences has been designed in view of the particular oligonucleotide sequence to which said spacers were to be attached to, and in order to provide a probe which can form a hairpin loop. Such an extended probe further carrying a fluorophore moiety at one terminus, and a quencher moiety at the other terminus, is useful a molecular beacon probe.

The use of an oligonucleotide as defined above as a probe or primer, for hybridizing with and optionally amplifying a nucleic acid from a hepatitis B virus (HBV) is also within the scope of the invention.

In that respect, the present invention further provides a first set of oligonucleotides, used as primers, and consisting of an oligonucleotide that consists of SEQ ID NO:2, and at least an oligonucleotide selected from the group consisting of an oligonucleotide that consists of SEQ ID NO:3, SEQ ID NO:4, SEQ ID NO:5, SEQ ID NO:6 and SEQ ID NO:7. In particular, said first set of oligonucleotides may consist of an oligonucleotide that consists of SEQ ID NO:2, an oligonucleotide that consists of SEQ ID NO:3, an oligonucleotide that consists of SEQ ID NO:4, an oligonucleotide that consists of SEQ ID NO:5, an oligonucleotide that consists of SEQ ID NO:6 and an oligonucleotide that consists of SEQ ID NO:7. Preferably said first set of oligonucleotide consists of, (i) an oligonucleotide that consists of SEQ ID NO:2, and an oligonucleotide that consists of SEQ ID NO:3, or (ii) an oligonucleotide that consists of SEQ ID NO:2, and an oligonucleotide that consists of SEQ ID NO:4, or (iii) an oligonucleotide that consists of SEQ ID NO:2, and an oligonucleotide that consists of SEQ ID NO:5, or (iv) an oligonucleotide that consists of SEQ ID NO:2, and an oligonucleotide that consists of SEQ ID NO:6, or (v) an oligonucleotide that consists of SEQ ID NO:2, and an oligonucleotide that consists of SEQ ID NO:7, or (vi) an oligonucleotide that consists of SEQ ID NO:2, an oligonucleotide that consists of SEQ ID NO:4 and an oligonucleotide that consists of SEQ ID NO:5, or (vii) an oligonucleotide that consists of SEQ ID NO:2, an oligonucleotide that consists of SEQ ID NO:6, and an oligonucleotide that consists of SEQ ID NO:7.

Still preferably, said first set of oligonucleotides, used as primers, consists in SEQ ID NO:2 and SEQ ID NO:3; SEQ ID NO:2 and SEQ ID NO:4; SEQ ID NO:2 and SEQ ID NO:5; SEQ ID NO:2, SEQ ID NO:4 and SEQ ID NO:5; SEQ ID NO:2 and SEQ ID NO:6; SEQ ID NO:2 and SEQ ID NO:7; or SEQ ID NO:2, SEQ ID NO:6 and SEQ ID NO:7

A further subject of the invention is a second set of oligonucleotides, useful for amplifying and detecting HBV nucleic acid, that comprises :
a) a set of oligonucleotides consisting of (i) an oligonucleotide that consists of SEQ ID NO:2, and an oligonucleotide that consists of SEQ ID NO:3, or (ii) an oligonucleotide that consists of SEQ ID NO:2, and an oligonucleotide that consists of SEQ ID NO:4, or (iii) an oligonucleotide that consists of SEQ ID NO:2, and an oligonucleotide that consists of SEQ ID NO:5, or (iv) an oligonucleotide that consists of SEQ ID NO:2, and an oligonucleotide that consists of SEQ ID NO:6, (v) an oligonucleotide that consists of SEQ ID NO:2, and an oligonucleotide that consists of SEQ ID NO:7, (vi) an oligonucleotide that consists of SEQ ID NO:2, an oligonucleotide that consists of SEQ ID NO:4 and an oligonucleotide that consists of SEQ ID NO:5, or (vii) an oligonucleotide that consists of SEQ ID NO:2, an oligonucleotide that consists of SEQ ID NO:6 and an oligonucleotide that consists of SEQ ID NO:7;
b) an oligonucleotide that consists of SEQ ID NO:8, SEQ ID NO:9, SEQ ID NO:10, SEQ ID NO:11, or their complementary sequence.

The invention further provides a method for specifically detecting a HBV by amplification in a biological sample, which method comprises the steps consisting of:
a) contacting a first set of oligonucleotides, used as primers, as defined above, with a biological sample or a nucleic acid preparation obtained from a biological sample under conditions suitable for the oligonucleotides to hybridize to a HBV nucleic acid present in the sample;
b) amplifying said HBV nucleic acid by polymerase chain reaction using said oligonucleotides as primers
c) detecting the amplification product, indicative of the presence of a HBV in the biological sample.

Preferably, the detection of said amplification product is performed by using an oligonucleotide that consists of SEQ ID NO:8, SEQ ID NO:9, SEQ ID NO:10, SEQ ID NO:11, or their complementary sequence, that is detectably labelled, as a probe. Such probe may be introduced at any step. Conveniently it is present or added to the mixture of primers.

The amplification method generally comprises the steps consisting of:
a) hybridizing at least one oligonucleotide primer as defined above to a template DNA consisting of a HBV DNA likely to be present in a biological sample;
b) carrying out a primer extension reaction to give a primer extension product;
c) denaturing the resulting DNA duplex to separate the primer extension product from the template HBV DNA; the primer extension product functioning as the other template DNA for the other primer, and
d) repeating a cycle of simultaneous primer extension reaction with two oligonucleotide primers, separation of the primer extension products from the DNA templates, and hybridization of primers to amplify a region of the target DNA.

Said biological sample may be of any type, *e.g.* a biological fluid, such as blood, serum, plasma or a tissue sample, such as obtainable by a liver biopsy. More preferably, the biological sample is serum or plasma.

The diagnostic method of the invention may be performed on a nucleic acid (*e.g.* DNA) preparation obtained from such biological sample, for instance by standard extraction procedures.

The invention further provides a kit for amplifying HBV in a biological sample, which kit comprises:
- at least a first set of oligonucleotides according to the invention, useful as primers; and
- means for amplifying a HBV nucleic acid.

Means for amplification may include for instance a thermostable DNA polymerase, dNTP solutions, MgCl₂. Uracyl DNA-Glycosylase may further be used to prevent PCR contamination.

In addition, the kit may further comprise a negative control (i.e. a sample free from HBV nucleic acid), a positive control (*i.e.* a double strain nucleic acid sequence of the HBV region to be amplified, cloned or not) and an internal control (i.e. a double strain nucleic acid sequence added to the sample and which detection after amplification can be differentiated from the target).

The kit may advantageously further comprises a probe as defined above. For instance, it may comprise a synthetic oligonucleotide that consists of SEQ ID NO:8, SEQ ID NO:9, SEQ ID NO:10, SEQ ID NO:11, or their complementary sequence, detectably labelled and useful as a probe.

The present invention will be further illustrated by the following figures and examples.

The one skilled in the art will readily understand that the invention encompasses oligonucleotides harboring sequence modifications as compared to the above described oligonucleotides (*i.e.* deletion, addition and/or substitution of a limited number of nucleotides), provided that such modifications are not detrimental to the sensitivity and/or specificity of HBV detection achieved using said oligonucleotide.

### FIGURES

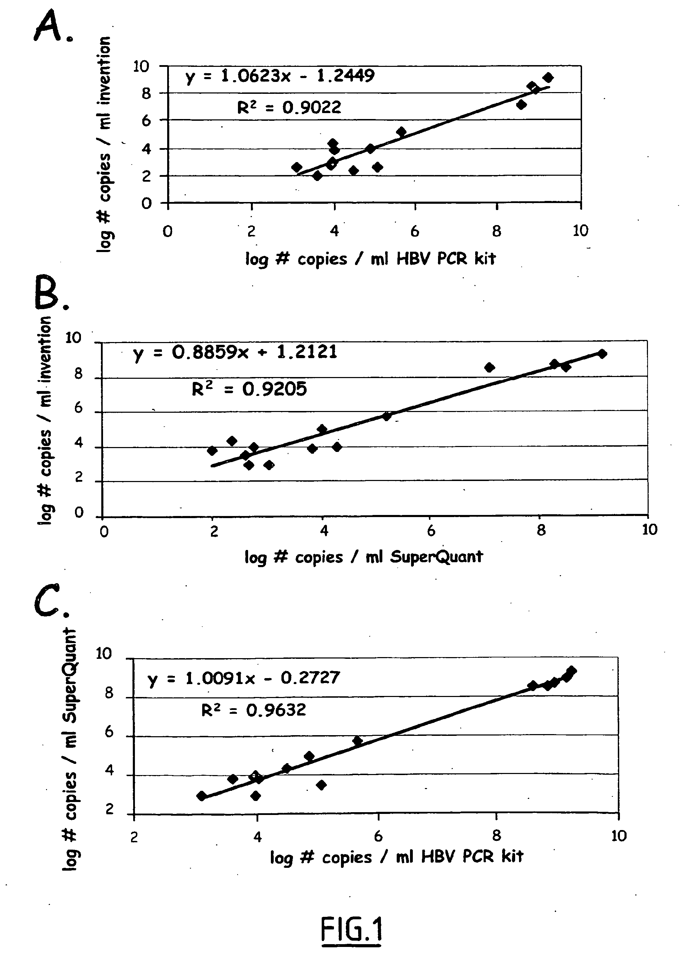

Figure 1 is a diagrammatic representation of pair comparisons of the quantification results provided by the test according to the invention (with SEQ ID N°2 and SEQ ID N°3 as primers and SEQ ID N°12 as probe), by HBV PCR kit Roche, and HBV NGI SuperQuant™ LabCorp. A: invention vs HBV PCR kit Roche; B: invention vs HBV NGI SuperQuant LabCorp; C: HBV NGI SuperQuant LabCorp vs HBV PCR kit Roche.

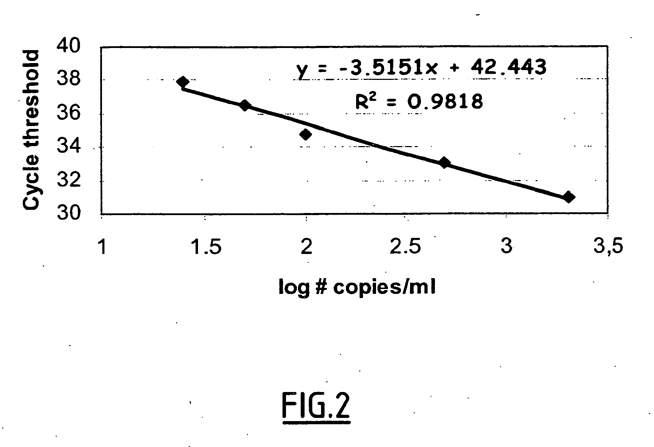

Figure 2 is a diagrammatic representation of the linearity of the detection signal against the number of copies/ml of extracted diluted Accurun™ HBV 325 DNA positive control by the test according to the invention (with SEQ ID N°2 and SEQID N°3 as primers and SEQ ID N°12 as probe).

### EXAMPLE : Specific detection of HBV by "real-time" PCR

### Materials and Methods :

### 1) Samples :

TeraPro™ Hepatitis B genotype Panel (Teragenix, catalog HBVGTP) : 15 positive HBV plasma samples (n° 1 to 15) from 15 different patients of various genotypes (3A, 1 B, 5C, 1 D, 3E, 2F).

TeraPro™ Hepatitis B Worldwide Genotype Panel (Teragenix, catalog HBVGTP-002a) : one positive HBV plasma sample of genotype G (n°50).

All these samples have been genotyped with the Visible Genetics kit (Trugene) and quantified by the HBV DNA kit Roche and the HBV DNA kit NGI (National Genetics Institute) SuperQuant.

Quantitation standard curve : use of extracted dilutions of panel Accurun 325 HBV DNA Positive control (BBI) or of sample n°15 from the TeraPro™ Hepatitis B Genotype Panel (Teragenix).

### 2) Extraction :

DNA was extracted from plasma samples using QiaAmp.DNA Blood mini kit, ref Qiagen 51104 (200 µl of sample used) or QIAamp MinElute virus vacuum kit, ref Qiagen 57714 (500 µl of sample used) according to the manufacturer recommendations.

### 3) Amplification:

Real-time PCR was performed using the Platinium quantitative PCR SuperMix UDG (Invitrogen 11730-017) : mix 2X that contains 60 U/ml Platinium Taq DNA Pol, 40 mM tris-HCl pH 8.4, 100 mM KCI, 6 mM MgCl₂, and 400 µM dGTP. To mix 1 X are added 1.5 µM primer SEQ ID N°2, 0.3 µM primer SEQ ID N°3 (or 0.3 µM primer SEQ ID N°4), 0.2 µM molecular beacon Fam-DarkQuencher probe SEQ ID N°12 (Eurogentec) and 3 mM MgCl₂ (to achieve 6 mM MgCl₂). Extracted DNA samples are then added to the mix containing primers, probe and additional MgCl2

The steps for the amplification were as follows :
- 1 x 2 minutes, 50°C (UDG is acting)
- 1 x 2 minutes, 95°C (UDG is inactivated ; Platinium Taq DNA Pol is activated)
- 50 x (15 seconds, 94°C - 30 seconds, 55°C - 30 seconds, 72°C)
- 4°C

### 4) Interpretation of results

For each assay is determined a threshold cycle (Ct) which is the level of fluorescence that is considered to be significantly above the background level of fluorescence measured in the early cycles of the amplification. A reference standard curve is constructed by plotting the log of known target concentrations against the corresponding Ct. The concentration of an unknown sample is then defined by mapping the corresponding Ct to the standard curve.

### Results

### 1) Comparison with existing PCR-based detection methods

14 samples (n° 1 to 14) from the TeraPro™ Hepatitis B genotype panel (Teragenix) diluted in plasma and extracted with the QiaAmp DNA Blood kit were quantified with either the test of the invention (with SEQ ID N°2 and SEQ ID N°3 as primers, and SEQ ID N°12 as molecular beacon probe) or with two existing marketed PCR-based detection tests : HBV PCR kit from Roche (assay developed to quantify until 2 10⁹ copies/ml) and HBV NGI SuperQuant kit from LabCorp (results communicated by Teragenix).

Primers and probe of the HBV PCR kit from Roche are selected in a conserved segment of the precore-core region whereas primers and probe of the HBV NGI SuperQuant kit from LabCorp are selected in a conserved segment of the polymerase gene.

**Table 1:**

| Sample | | | HBV PCR kit Roche | | invention | | HBV NGI SuperQuant™ LabCorp | |
|---|---|---|---|---|---|---|---|---|
| # | Genotype (Visible Genetics results) | Serotype | # copies/ml | Log # copies/ml | # copies/ml | log # copies/ml | # copies/ml | log # copies/ml |
| 1 | A | adw2 | 3.95 10⁸ | 8,60 | 1, 34.10⁷ | 7,12 | 3 10⁸ | 8,48 |
| 2 | A | adw2 | 3.110⁴ | 4,49 | 263 | 2,34 | 2,310⁴ | 4,36 |
| 3 | A | adw2 | 9.710³ | 3,99 | 1,1 10³ | 3,02 | 1,1 10³ | 3 |
| 4 | B | adw2 | 4.610⁵ | 5,66 | 1,66.10⁵ | 5,21 | 5,510⁵ | 5,74 |
| 5 | E | adw2 | 8.710³ | 3,94 | 566 | 2,75 | 8,810³ | 3,94 |
| 6 | C | adr | 8.5 10⁸ | 8,93 | 2.10⁸ | 8,3 | 4,910⁸ | 8,69 |
| 7 | C | adr | 1.1510⁵ | 5,06 | 399 | 2,59 | 3,110³ | 3,49 |
| 8 | C | adr | 6.7 10⁸. | 8,83 | 3,15.10⁸ | 8,49 | 3,4 10⁸ | 8,53 |
| 9 | C | ayw2 | 1.2 10³ | 3,08 | 494 | 2,65 | 10³ | 3 |
| 10 | C | ayw2 | 4 10³ | 3,60 | 101 | 2 | 6,9 10³ | 3,83 |
| 11 | D | ayw2 | 1.64 10⁹ | 9,21 | 1,52.10⁹ | 9,17 | 2 10⁹ | 9,3 |
| 12 | E | ayw4 | 7.610⁴ | 4,88 | 1,01.10⁴ | 4 | 9,7 10⁴ | 4,98 |
| 13 | E | ayw4 | 9.7 10³ | 3,99 | 2.10⁴ | 4,27 | 10⁴ | 4 |
| 14 | F | ayw4 | 10.6 10³ | 4,03 | 6,1 10³ | 3,82 | 7,4 10³ | 3,86 |
| 50 | G | ND | > 2 10⁵ * | > 5.3 | 1.66 10⁷ | 7.2 | 2.9 10⁷ | 7.46 |
| 15 | F | ayw4 | 1.35 10⁹ | 9,13 | Used for calibration | | 8,5 10⁸ | 8,92 |

| | | | | | | | | |
|---|---|---|---|---|---|---|---|---|
| * this sample has been quantified on the Cobas Amplicor HBV Monitor kit with a range of quantification of 200 to 2 10⁵ copies/ml | | | | | | | | |

The test according to the invention is able to specifically detect and quantify HBV genotypes A to G.

Overall pair comparison of the results obtained with the three tests indicates that the results provided by the test according to the invention show a good correlation with that provided with each of the HBV PCR kit from Roche, or the HBV NGI SuperQuant™ from LabCorp (figure 1).

### 2) Sensitivity of detection

### a) Linearity of the quantification range

To demonstrate that the detection signal (cycle threshold) as obtained with the test of the invention (with SEQ ID N°2 and SEQ ID N°3 as primers, and SEQ ID N°12 as molecular beacon probe) correlates with the number of HBV DNA copies per ml, sample number 15 from the TeraPro™ HBV genotype panel (Teragenix) was diluted in plasma and extracted with the QiaAmp DNA Blood kit. The cycle threshold measured with increasing dilutions of the sample until the detection limit (100 copies/ml) is reported in Table 2 below. The linear relationship between the number of copies and the cycle threshold is illustrated in figure 2.

**Table 2**

| Panel TERAGENIX N°15 | | Threshold Cycle (Ct) | | | | | | |
|---|---|---|---|---|---|---|---|---|
| copies/ml | Log copies/ml | 1 | 2 | 3 | Mean Ct | SD* | CV^{¤} | RFU^{$} |
| 10⁹ | 9 | 14,7 | 14,2 | 14,6 | 14,5 | 0,26 | 1,82% | 2750/3000 |
| 10⁸ | 8 | 18,0 | 17,7 | 17,7 | 17,8 | 0,17 | 0,97% | 2500/3250 |
| 10⁷ | 7 | 20,8 | 21,0 | 21,3 | 21,0 | 0,25 | 1,20% | 2500/2750 |
| 10⁶ | 6 | 23,4 | 25,1 | 24,6 | 24,4 | 0,87 | 3,59% | 2250/2400 |
| 10⁵ | 5 | 27,8 | 27,8 | 27,6 | 27,7 | 0,12 | 0,42% | 2000 |
| 10⁴ | 4 | 30,2 | 30,4 | 31,5 | 30,7 | 0,70 | 2,28% | 1500/1700 |
| 10³ | 3 | 34,3 | 35,3 | 33,8 | 34,5 | 0,76 | 2,22% | 1000/1250 |
| 100 | 2 | 38,0 | 37,1 | 37,2 | 37,4 | 0,49 | 1,32% | 600/750 |

| | | | | | | | | |
|---|---|---|---|---|---|---|---|---|
| * Standard deviation ¤ Coefficient of variation in % $ Relative Fluorescence Unit | | | | | | | | |

On HBV positive samples extracted with the QiaAmp DNA Blood kit from Qiagen, the test according to the invention is able to quantify HBV from 100 to 10⁹ copies/ml with a good correlation coefficient (0.998) when the log of the copy number is plotted versus the threshold cycle (Ct).

### b) Sensitivity of detection

The Accurun™ HBV 325 DNA positive control (Boston Biomedica) was used as a reference sample to determine the limit of sensitivity of the test of the invention (with SEQ ID N°2 and SEQ ID N°3 as primers, and SEQ ID N°12 as molecular beacon probe). To that end, the sample was diluted into plasma and DNA was extracted with the QlAamp MinElute extraction kit, as explained above. The results of this analysis, reported in the Table 3 below, show that the test according to the invention made it possible to detect as low as 25 copies/ml of the Accurun™ HBV reference on HBV positive samples extracted with the QIAamp MinElute extraction kit from Qiagen.

**Table 3:**

| Accurun HBV | | Cycle Threshold (Ct) | | | | | |
|---|---|---|---|---|---|---|---|
| # copies/ml | Log # | 1 | 2 | mean Ct | SD* | CV^{¤} | RFU^{$} |
| 2000 | 3,3 | 31,4 | 30,6 | 31,0 | 0,40 | 1,29% | 500/360 |
| 500 | 2,7 | 33,5 | 32,4 | 33,0 | 0,55 | 1,67% | 330/375 |
| 100 | 2 | 35,0 | 34,5 | 34,8 | 0,25 | 0,72% | 250/300 |
| 50 | 1,7 | 36,6 | 36,4 | 36,5 | 0,10 | 0,27% | 150/175 |
| 25 | 1,4 | 37,8 | 37,9 | 37,9 | 0,05 | 0,13% | 80/150 |
| 10 | 1 | N/A | N/A | | | | 0 |

| | | | | | | | |
|---|---|---|---|---|---|---|---|
| * Standard deviation ¤ Coefficient of variation in % $ Relative Fluorescence Unit | | | | | | | |

The mean limit of sensitivity was further assayed in the same conditions on each of 15 samples from the TeraPro™ HBV genotype panel and on the sample n° 50 from the TeraProTM Hepatitis B Worldwide Genotype Panel as summarized in Table 4.

**Table 4**

| Sample | Genotype (Visible Genetics results) | Cycle threshold | log # copies/ml | Estimated # copies/ml |
|---|---|---|---|---|
| 1 | A | 36,6 | 1,66 | 46 |
| 2 | A | 36,7 | 1,63 | 43 |
| 3 | A | 41 | 0,48 | < 25 |
| 4 | B | 34,9 | 1,69 | 140 |
| 5 | E | 38,2 | 1,2 | 16 |
| 6 | C | 40 | 0,7 | < 25 |
| 7 | C | 39,2 | 0,9 | < 25 |
| 8 | C | 38,4 | 1,14 | 14 |
| 9 | C | 38,9 | 1 | < 25 |
| 10 | C | 40,2 | 0,7 | < 25 |
| 11 | D | 36,9 | 1,58 | 38 |
| 12 | E | 38,4 | 1,14 | 14 |
| 13 | E | 40,1 | 0,7 | < 25 |
| 14 | F | 38.6 | 1.08 | 12 |
| 15 | F | 37.9 | 1.3 | 20 |
| 50 | G | 37.6 | 1.3 | 20 |
| Mean sensitivity limit | | | | < 32 |

Therefore, the mean sensitivity limit of the test of the invention as obtained with the TeraPro™ HBV genotype panel diluted in plasma and extracted with QiaAmp Min Elute virus vacuum kit was found to be very close to the mean sensitivity limit obtained with the Accurun™ HBV 325 DNA positive control (detection limit < 32 copies /ml compared with 25 copies/ml).

The sensitivity limit of the test according to the invention is improved compared with the PCR-based detection tests on the market, such as the Cobas Amplicor HBV Monitor kit from Roche Diagnostics (sensitivity limit of 200 copies/ml) or the Hepatitis B virus NGI SuperQuant kit from LabCorp (sensitivity limit of 100 copies/ml).

### 3) Comparison of the set of primers SEQ ID N°2 and SEQ ID N°3 with

### the set of primers SEQ ID N°2 and SEQ ID N°4

For this comparison, dilutions of Accurun 325 HBV DNA positive control in plasma, extracted with the QiaAmp DNA blood mini kit, were used. Results of threshold cycles (Ct) are reported in table 5.

**Table 5 :**

| Accurun HBV | | SEQ ID N°2 + SEQ ID N°3 | | | | SEQ ID N°2 + SEQ ID N°4 | | | |
|---|---|---|---|---|---|---|---|---|---|
| # copies/ml | Log # copies/ml | Ct (1) | Ct (2) | Mean Ct | Mean RFUs | Ct (1) | Ct (2) | mean Ct | Mean RFUs |
| 10⁵ | 5 | 28.9 | 28.9 | 28.9 | 625 | 27.2 | 26.0 | 26.6 | 550 |
| 10⁴ | 4 | 31.6 | 31.6 | 31.6 | 500 | 30.0 | 31.6 | 30.8 | 350 |
| 10³ | 3 | 34.3 | 34.8 | 34.6 | 350 | 33.3 | 311.9 | 32.6 | 225 |
| 10² | 2 | 37.5 | ND | 37.5 | 200 | 37.8 | 37.7 | 37.8 | 100 |

Both sets of primers amplified the extracted dilutions of Accurun HBV with a same limit of sensitivity (10² copies/ml), but a better reproducibility of threshold cycle and a better correlation were obtained with the set of primers SEQ ID N°2 and SEQ ID N°3 (correlation coefficient of 0.999) than with the set of primers SEQ ID N°2 and SEQ ID N°4 (correlation coefficient of 0.970).

### REFERENCES

- Ausubel et al. (eds.), Current Protocols in Molecular Biology, John Wiley & Sons, Inc. (1994)
- Cayouette M, Sucharzuk A, Moores J, Tyagi S, and Kramer FR (1999) Using molecular beacons to monitor PCR product formation. Strategies Newsl. 12:85-88.
- Fahy et al. (1991) PCR Meth. Appl., 1, 25-33
- Kwoh et al. (1989) PNAS, 86, 1173-1177
- Perbal, A Practical Guide To Molecular Cloning (1984)
- Saiki et al., (1988), Science, 239:487
- Sambrook, Fritsch & Maniatis, Molecular Cloning: A Laboratory Manual, Second Edition (1989) Cold Spring Harbor Laboratory Press, Cold Spring Harbor, New York
- Tyagi S and Kramer FR (1996) Nature Biotechnol., 16, 303-308
- Walker et al. (1992) P.N.A.S, 89, 392-396
- Yaron et al., (1979) Analytical Biochemistry 95: 228-235

## Claims

1. An oligonucleotide consisting in a sequence selected from the group consisting of SEQ ID NO:2, SEQ ID NO:3, SEQ ID NO:4, SEQ ID NO:6, SEQ ID NO:7 and their complementary sequences.

2. The oligonucleotide according to claim 1, which consists of SEQ ID NO:2 or its complementary sequence.

3. The oligonucleotide according to claim 1, which consists of SEQ ID NO: 3, or its complementary sequence.

4. The oligonucleotide according to claim 1, which consists of SEQ ID NO: 4, or its complementary sequence.

5. The oligonucleotide according to claim 1, which consists of SEQ ID NO: 6, or its complementary sequence.

6. The oligonucleotide according to claim 1, which consists of SEQ ID NO: 7, or its complementary sequence.

7. Use of an oligonucleotide as defined in any of claims 1 to 6, as a probe or primer, for hybridizing with and amplifying a nucleic acid from a hepatitis B virus (HBV).

8. Use of an oligonucleotide consisting of a sequence selected from the group consisting of SEQ ID NO:8, SEQ ID NO:9, SEQ ID NO:10, SEQ ID NO:11, and their complementary sequence, optionally linked on the 5' and/or 3' terminus by additional sequences unable to hybridize with HBV nucleic acids under standard hybridization conditions, as a probe for hybridizing with a nucleic acid from HBV.

9. The use according to claim 8, wherein said oligonucleotide consists of a sequence selected from the group consisting of SEQ ID NO:8, SEQ ID NO:9, SEQ ID NO:10, SEQ ID NO:11; and their complementary sequence.

10. The use according to claim 8, wherein said oligonucleotide includes a sequence selected from the group consisting of SEQ ID NO:8, SEQ ID NO:9, SEQ ID NO:10, SEQ ID NO:11, and their complementary sequence and carries a fluorophore moiety at one terminus, and a quencher moiety at the other terminus.

11. The use according to claim 10, wherein said oligonucleotide consists of a sequence selected from the group consisting of SEQ ID NO:12, SEQ ID NO:13, SEQ ID NO:14 and SEQ ID NO:15, and carries a fluorophore moiety at one terminus, and a quencher moiety at the other terminus.

12. A set of oligonucleotides consisting of an oligonucleotide that consists in SEQ ID NO:2, and at least an oligonucleotide selected from the group consisting of an oligonucleotide that consists in SEQ ID NO:3, SEQ ID NO:4, SEQ ID NO:5, SEQ ID NO:6 and SEQ ID NO:7.

13. A set of oligonucleotides according to claim 12, which consists of:
(i) an oligonucleotide that consists in SEQ ID NO:2, and an oligonucleotide that consists in SEQ ID NO:3;
(ii) an oligonucleotide that consists in SEQ ID NO:2, and an oligonucleotide that consists in SEQ ID NO:4;
(iii) an oligonucleotide that consists in SEQ ID NO:2, and an oligonucleotide that consists in SEQ ID NO:5;
(iv) an oligonucleotide that consists in SEQ ID NO:2, and an oligonucleotide that consists in SEQ ID NO:6;
(v) an oligonucleotide that consists in SEQ ID NO:2, and an oligonucleotide that consists in SEQ ID NO:7;
vi) an oligonucleotide that consists in SEQ ID NO:2, an oligonucleotide that consists in SEQ ID NO:4 and an oligonucleotide that consists in SEQ ID NO:5; and
(vii) an oligonucleotide that consists in SEQ ID NO:2, an oligonucleotide that consists in SEQ ID NO:6 and an oligonucleotide that includes SEQ ID NO:7.

14. A set of oligonucleotides comprising:
a) a set of oligonucleotides according to claim 12 or 13; and
b) an oligonucleotide that includes a sequence selected from the group consisting of SEQ ID NO:8, SEQ ID NO:9, SEQ ID NO:10, SEQ ID NO:11, and their complementary sequence.

15. A set of oligonucleotides according to claim 14, that comprises:
a) a set of oligonucleotides according to claim 12 or 13; and
b) an oligonucleotide that consists of a sequence selected from the group consisting of SEQ ID NO:12, SEQ ID NO:13, SEQ ID NO:14 and SEQ ID NO:15, and carries a fluorophore moiety at one terminus, and a quencher moiety at the other terminus.

16. A method for specifically detecting a HBV by amplification in a biological sample, which method comprises the steps consisting of:
a) contacting a set of oligonucleotides according to claim 12 or 13 with a biological sample or nucleic acid preparation obtained from a biological sample, under conditions suitable for the oligonucleotides to hybridize to a HBV nucleic acid present in the sample;
b) amplifying said HBV nucleic acid using said oligonucleotides as primers;
c) detecting the amplification product, indicative of the presence of a HBV in the biological sample.

17. The method according to claim 16, wherein HBV nucleic acid is amplified by polymerase chain reaction.

18. The method according to claim 16 or 17, wherein the detection of said amplification product is performed by using an oligonucleotide that includes a sequence selected from the group consisting of SEQ ID NO:8, SEQ ID NO:9, SEQ ID NO:10, SEQ ID NO:11, and their complementary sequence, and that is detectably labelled, as a probe.

19. The method according to claim 18, wherein said oligonucleotide that includes a sequence selected from the group consisting of SEQ ID NO:8, SEQ ID NO:9, SEQ ID NO:10, SEQ ID NO:11, and their complementary sequence, and carries a fluorophore moiety at one terminus, and a quencher moiety at the other terminus.

20. The method according to claim 18 or 19, wherein said oligonucleotide that includes a sequence selected from the group consisting of SEQ ID NO:8, SEQ ID NO:9, SEQ ID NO:10, SEQ ID NO:11, and their complementary sequence, is SEQ ID NO:12, SEQ ID NO:13, SEQ ID NO:14 or SEQ ID NO:15.

21. A kit for amplifying HBV in a biological sample, which kit comprises :
- at least a set of oligonucleotides according to claim 12 or 13, useful as primers;
- means for amplifying a HBV nucleic acid.

22. The kit according to claim 21, which further comprises means for the detection of the amplified product.

23. The kit according to claim 21 or 22, wherein the means for amplifying HBV nucleic acid are means for amplification by Polymerase Chain Reaction.

24. The kit according to any of claims 21 to 23, which comprises an oligonucleotide that includes a sequence selected from the group consisting of SEQ ID NO:8, SEQ ID NO:9, SEQ ID NO:10, SEQ ID NO:11, and their complementary sequence, detectably labelled and useful as a probe.

25. The kit according to claim 24, wherein said oligonucleotide that includes a sequence selected from the group consisting of SEQ ID NO:8, SEQ ID NO:9, SEQ ID NO:10, SEQ ID NO:11, and their complementary sequence, is SEQ ID NO:12, SEQ ID NO:13, SEQ ID NO:14 or SEQ ID NO:15.

## Patentansprüche

1. Oligonucleotid, das aus einer Sequenz, die aus der Gruppe ausgewählt ist, die aus SEQ ID NR. 2, SEQ ID NR. 3, SEQ ID NR. 4, SEQ ID NR. 6 und SEQ ID NR. 7 besteht, und deren komplementären Sequenzen besteht.

2. Oligonucleotid nach Anspruch 1, das aus der SEQ ID NR. 2 oder ihrer komplementären Sequenz besteht.

3. Oligonucleotid nach Anspruch 1, das aus der SEQ ID NR. 3 oder ihrer komplementären Sequenz besteht.

4. Oligonucleotid nach Anspruch 1, das aus der SEQ ID NR. 4 oder ihrer komplementären Sequenz besteht.

5. Oligonucleotid nach Anspruch 1, das aus der SEQ ID NR. 6 oder ihrer komplementären Sequenz besteht.

6. Oligonucleotid nach Anspruch 1, das aus der SEQ ID NR. 7 oder ihrer komplementären Sequenz besteht.

7. Verwendung eines Oligonucleotids wie in einem der Ansprüche 1 bis 6 definiert als Sonde oder Primer für eine Hybridisierung mit einer Nucleinsäure aus einem Hepatitis-B-Virus (HBV) und deren Amplifizierung.

8. Verwendung eines Oligonucleotids, das aus einer Sequenz, die aus der Gruppe ausgewählt ist, die aus SEQ ID NR. 8, SEQ ID NR. 9, SEQ ID NR. 10 und SEQ ID NR. 11 besteht, und deren komplementärer Sequenz, wahlweise mit dem 5'- und/oder 3'-Terminus durch weitere Sequenzen, die nicht in der Lage sind, unter Standardhybridisierungsbedingungen mit HBV-Nucleinsäuren zu hybridisieren, verbunden, besteht, als Sonde für die Hybridisierung mit einer Nucleinsäure von HBV.

9. Verwendung nach Anspruch 8, wobei das Oligonucleotid aus einer Sequenz, die aus der Gruppe ausgewählt ist, die aus SEQ ID NR. 8, SEQ ID NR. 9, SEQ ID NR. 10 und SEQ ID NR. 11 besteht, und deren komplementärer Sequenz besteht.

10. Verwendung nach Anspruch 8, wobei das Oligonucleotid eine Sequenz, die aus der Gruppe ausgewählt ist, die aus SEQ ID NR. 8, SEQ ID NR. 9, SEQ ID NR. 10 und SEQ ID NR. 11 besteht, und ihre komplementäre Sequenz umfasst und eine Fluorophorgrundeinheit an einem Terminus und eine Quencher-Grundeinheit an dem anderen Terminus trägt.

11. Verwendung nach Anspruch 10, wobei das Oligonucleotid aus einer Sequenz, die aus der Gruppe ausgewählt ist, die aus SEQ ID NR. 12, SEQ ID NR. 13, SEQ ID NR. 14 und SEQ ID NR. 15 besteht, besteht und eine Fluorophorgrundeinheit an einem Terminus und eine Quencher-Grundeinheit an dem anderen Terminus trägt.

12. Satz von Oligonucleotiden, der aus einem Oligonucleotid, das aus SEQ ID NR. 2 besteht, und mindestens einem Oligonucleotid besteht, das aus der Gruppe ausgewählt ist, die aus einem Oligonucleotid besteht, das aus SEQ ID NR. 3, SEQ ID NR. 4, SEQ ID NR. 5, SEQ ID NR. 6 und SEQ ID NR. 7 besteht.

13. Satz von Oligonucleotiden nach Anspruch 12, der aus:
(i) einem Oligonucleotid, das aus SEQ ID NR. 2 besteht, und einem Oligonucleotid, das aus SEQ ID NR. 3 besteht,
(ii) einem Oligonucleotid, das aus SEQ ID NR. 2 besteht, und einem Oligonucleotid, das aus SEQ ID NR. 4 besteht,
(iii) einem Oligonucleotid, das aus SEQ ID NR. 2 besteht, und einem Oligonucleotid, das aus SEQ ID NR. 5 besteht,
(iv) einem Oligonucleotid, das aus SEQ ID NR. 2 besteht, und einem Oligonucleotid, das aus SEQ ID NR. 6 besteht,
(v) einem Oligonucleotid, das aus SEQ ID NR. 2 besteht, und einem Oligonucleotid, das aus SEQ ID NR. 7 besteht,
(vi) einem Oligonucleotid, das aus SEQ ID NR. 2 besteht, einem Oligonucleotid, das aus SEQ ID NR. 4 besteht, und einem Oligonucleotid, das aus SEQ ID NR. 5 besteht, und
(vii) einem Oligonucleotid, das aus SEQ ID NR. 2 besteht, einem Oligonucleotid, das aus SEQ ID NR. 6 besteht, und einem Oligonucleotid, das SEQ ID NR. 7 umfasst, besteht.

14. Satz von Oligonucleotiden, der:
a) einen Satz von Oligonucleotiden nach Anspruch 12 oder 13 und
b) ein Oligonucleotid, das eine Sequenz, die aus der Gruppe ausgewählt ist, die aus SEQ ID NR. 8, SEQ ID NR. 9, SEQ ID NR. 10 und SEQ ID NR. 11 besteht, und ihre komplementäre Sequenz umfasst,
umfasst.

15. Satz von Oligonucleotiden nach Anspruch 14, der:
a) einen Satz von Oligonucleotiden nach Anspruch 12 oder 13 und
b) ein Oligonucleotid, das aus einer Sequenz, die aus der Gruppe ausgewählt ist, die aus SEQ ID NR. 12, SEQ ID NR. 13, SEQ ID NR. 14 und SEQ ID NR. 15 besteht, besteht und eine Fluorophorgrundeinheit an einem Terminus und eine Quencher-Grundeinheit an dem anderen Terminus trägt,
umfasst.

16. Verfahren zum spezifischen Detektieren eines HBV durch Amplifikation in einer biologischen Probe, das die Stufen umfasst, die aus dem:
a) In-Berührung-Bringen eines Satzes von Oligonucleotiden nach Anspruch 12 oder 13 mit einer biologischen Probe oder einem aus einer biologischen Probe erhaltenen Nucleinsäurepräparat unter Bedingungen, die für die Oligonucleotide geeignet sind, mit einer in der Probe vorhandenen HBV-Nucleinsäure zu hybridisieren,
b) Amplifizieren dieser HBV-Nucleinsäure unter Verwendung dieser Oligonucleotide als Primer und
c) Detektieren des Amplifikationsprodukts, das ein Anzeichen für das Vorhandensein eines HBV in der biologischen Probe ist,
bestehen.

17. Verfahren nach Anspruch 16, wobei die HBV-Nucleinsäure durch die Polymerasekettenreaktion amplifiziert wird.

18. Verfahren nach Anspruch 16 oder 17, wobei die Detektion des Amplifikationsprodukts unter Verwendung eines Oligonucleotids, das eine Sequenz, die aus der Gruppe ausgewählt ist, die aus SEQ ID NR. 8, SEQ ID NR. 9, SEQ ID NR. 10 und SEQ ID NR. 11 besteht, und ihre komplementäre Sequenz umfasst und detektierbar markiert ist, als Sonde durchgeführt wird.

19. Verfahren nach Anspruch 18, wobei das Oligonucleotid eine Sequenz, die aus der Gruppe ausgewählt ist, die aus SEQ ID NR. 8, SEQ ID NR. 9, SEQ ID NR. 10 und SEQ ID NR. 11 besteht, und ihre komplementäre Sequenz umfasst und eine Fluorophorgrundeinheit an einem Terminus und eine Quencher-GrundeinheJet an dem anderen Terminus trägt.

20. Verfahren nach Anspruch 18 oder 19, wobei das Oligonucleotid, das eine Sequenz, die aus der Gruppe ausgewählt ist, die aus SEQ ID NR. 8, SEQ ID NR. 9, SEQ ID NR. 10 und SEQ ID NR. 11 besteht, und ihre komplementäre Sequenz umfasst, SEQ ID NR. 12, SEQ ID NR. 13, SEQ ID NR. 14 oder SEQ ID NR. 15 ist.

21. Kit für die HBV-Amplifikation in einer biologischen Probe, der:
- mindestens einen Satz von Oligonucleotiden nach Anspruch 12 oder 13, die als Primer nützlich sind, und
- Mittel für die Amplifikation einer HBV-Nucleinsäure
umfasst.

22. Kit nach Anspruch 21, der weiterhin Mittel für die Detektion des amplifizierten Produkts umfasst.

23. Kit nach Anspruch 21 oder 22, wobei die Mittel für die Amplifikation der HBV-Nucleinsäure Mittel für die Amplifikation durch Polymerasekettenreaktion sind.

24. Kit nach einem der Ansprüche 21 bis 23, der ein Oligonucleotid, das eine Sequenz, die aus der Gruppe ausgewählt ist, die aus SEQ ID NR. 8, SEQ ID NR. 9, SEQ ID NR. 10 und SEQ ID NR. 11 besteht, und ihre komplementäre Sequenz umfasst, detektierbar markiert und als Sonde geeignet ist, umfasst.

25. Kit nach Anspruch 24, wobei das Oligonucleotid, das eine Sequenz, die aus der Gruppe ausgewählt ist, die aus SEQ ID NR. 8, SEQ ID NR. 9, SEQ ID NR. 10 und SEQ ID NR. 11 besteht, und ihre komplementäre Sequenz umfasst, SEQ ID NR. 12, SEQ ID NR. 13, SEQ ID NR. 14 oder SEQ ID NR. 15 ist.

## Revendications

1. Oligonucléotide consistant en une séquence choisie dans le groupe comprenant la SEQ ID NO: 2, la SEQ ID NO: 3, la SEQ ID NO: 4, la SEQ ID NO: 6, la SEQ ID NO: 7 et leurs séquences complémentaires.

2. Oligonucléotide selon la revendication 1, qui consiste en la SEQ ID NO: 2 ou sa séquence complémentaire.

3. Oligonucléotide selon la revendication 1, qui consiste en la SEQ ID NO: 3, ou sa séquence complémentaire.

4. Oligonucléotide selon la revendication 1, qui consiste en la SEQ ID NO: 4, ou sa séquence complémentaire.

5. Oligonucléotide selon la revendication 1, qui consiste en la SEQ ID NO: 6, ou sa séquence complémentaire.

6. Oligonucléotide selon la revendication 1, qui consiste en la SEQ ID NO: 7, ou sa séquence complémentaire.

7. Utilisation d'un oligonucléotide tel que défini dans l'une quelconque des revendications 1 à 6, en tant que sonde ou amorce, dans le but de s'hybrider avec un acide nucléique provenant d'un virus de l'hépatite B (VHB) et de l'amplifier.

8. Utilisation d'un oligonucléotide consistant en une séquence choisie dans le groupe comprenant la SEQ ID NO: 8, la SEQ ID NO: 9, la SEQ ID NO: 10, la SEQ ID NO: 11, et leurs séquences complémentaires, éventuellement liées au niveau de leur extrémité 5' et/ou 3' à des séquences supplémentaires incapables de s'hybrider avec des acides nucléiques du VHB dans des conditions d'hybridation standards, en tant que sonde pour s'hybrider avec un acide nucléique provenant du VHB.

9. Utilisation selon la revendication 8, dans laquelle ledit oligonucléotide consiste en une séquence choisie dans le groupe comprenant la SEQ ID NO: 8, la SEQ ID NO: 9, la SEQ ID NO: 10, la SEQ ID NO: 11, et leur séquence complémentaire.

10. Utilisation selon la revendication 8, dans laquelle ledit oligonucléotide comprend une séquence choisie dans le groupe comprenant la SEQ ID NO: 8, la SEQ ID NO: 9, la SEQ ID NO: 10, la SEQ ID NO: 11, et leurs séquences complémentaires et porte un groupement fluorophore au niveau d'une extrémité, et un groupement quencher au niveau de l'autre extrémité.

11. Utilisation selon la revendication 10, dans laquelle ledit oligonucléotide consiste en une séquence choisie dans le groupe comprenant la SEQ ID NO: 12, la SEQ ID NO: 13, la SEQ ID NO: 14 et la SEQ ID NO: 15, et porte un groupement fluorophore au niveau d'une extrémité, et un groupement bloqueur au niveau de l'autre extrémité.

12. Ensemble d'oligonucléotides consistant en un oligonucléotide qui consiste en la SEQ ID NO: 2, et au moins un oligonucléotide choisi dans le groupe comprenant un oligonucléotide qui consiste en la SEQ ID NO: 3, la SEQ ID NO: 4, la SEQ ID NO: 5, la SEQ ID NO: 6 et la SEQ ID NO: 7.

13. Ensemble d'oligonucléotides selon la revendication 12, qui consiste en :
(i) un oligonucléotide qui consiste en la SEQ ID NO: 2, et un oligonucléotide qui consiste en la SEQ ID NO: 3 ;
(ii) un oligonucléotide qui consiste en la SEQ ID NO: 2, et un oligonucléotide qui consiste en la SEQ ID NO: 4 ;
(iii) un oligonucléotide qui consiste en la SEQ ID NO: 2, et un oligonucléotide qui consiste en la SEQ ID NO: 5 ;
(iv) un oligonucléotide qui consiste en la SEQ ID NO: 2, et un oligonucléotide qui consiste en la SEQ ID NO: 6 ;
(v) un oligonucléotide qui consiste en la SEQ ID NO: 2, et un oligonucléotide qui consiste en la SEQ ID NO: 7 ;
(vi) un oligonucléotide qui consiste en la SEQ ID NO: 2, un oligonucléotide qui consiste en la SEQ ID NO: 4 et un oligonucléotide qui consiste en la SEQ ID NO: 5 ; et
(vii) un oligonucléotide qui consiste en la SEQ ID NO: 2, un oligonucléotide qui consiste en la SEQ ID NO: 6 et un oligonucléotide qui comprend la SEQ ID NO: 7.

14. Ensemble d'oligonucléotides comprenant :
a) un ensemble d'oligonucléotides selon la revendication 12 ou 13 ; et
b) un oligonucléotide qui comprend une séquence choisie dans le groupe comprenant la SEQ ID NO: 8, la SEQ ID NO: 9, la SEQ ID NO: 10, la SEQ ID NO: 11, et leur séquence complémentaire.

15. Ensemble d'oligonucléotides selon la revendication 14, qui comprend :
a) un ensemble d'oligonucléotides selon la revendication 12 ou 13 ; et
b) un oligonucléotide qui consiste en une séquence choisie dans le groupe comprenant la SEQ ID NO: 12, la SEQ ID NO: 13, la SEQ ID NO: 14 et la SEQ ID NO: 15, et porte un groupement fluorophore au niveau d'une extrémité, et un groupement quencher au niveau de l'autre extrémité.

16. Procédé pour détecter spécifiquement un VHB par amplification dans un échantillon biologique, le procédé comprenant les étapes consistant à :
a) mettre en contact un ensemble d'oligonucléotides selon la revendication 12 ou 13 avec un échantillon biologique ou une préparation d'acides nucléiques obtenue à partir d'un échantillon biologique, dans des conditions appropriées pour que les oligonucléotides s'hybrident avec un acide nucléique de VHB présent dans l'échantillon ;
b) amplifier ledit acide nucléique de VHB en utilisant lesdits oligonucléotides comme amorces ;
c) détecter le produit de l'amplification, indicateur de la présence d'un VHB dans l'échantillon biologique.

17. Procédé selon la revendication 16, dans lequel l'acide nucléique de VHB est amplifié par réaction d'amplification en chaîne par polymérase.

18. Procédé selon la revendication 16 ou 17, dans lequel la détection dudit produit d'amplification est effectuée en utilisant un oligonucléotide qui comprend une séquence choisie dans le groupe comprenant la SEQ ID NO: 8, la SEQ ID NO: 9, la SEQ ID NO: 10, la SEQ ID NO: 11, et leurs séquences complémentaires, et qui est marqué de manière détectable, comme sonde.

19. Procédé selon la revendication 18, dans lequel ledit oligonucléotide comprend une séquence choisie dans le groupe comprenant la SEQ ID NO: 8, la SEQ ID NO: 9, la SEQ ID NO: 10, la SEQ ID NO: 11, et leur séquence complémentaire, et porte un groupement fluorophore au niveau d'une extrémité, et un groupement quencher au niveau de l'autre extrémité.

20. Procédé selon la revendication 18 ou 19, dans lequel ledit oligonucléotide qui comprend une séquence choisie dans le groupe comprenant la SEQ ID NO: 8, la SEQ ID NO: 9, la SEQ ID NO: 10, la SEQ ID NO: 11, et leur séquence complémentaire, est la SEQ ID NO: 12, la SEQ ID NO: 13, la SEQ ID NO: 14 ou la SEQ ID NO: 15.

21. Kit pour amplifier le VHB dans un échantillon biologique, ledit kit comprenant :
- au moins un ensemble d'oligonucléotides selon la revendication 12 ou 13, utiles comme amorces ;
- un moyen pour amplifier un acide nucléique de VHB.

22. Kit selon la revendication 21, qui comprend en outre un moyen pour détecter le produit amplifié.

23. Kit selon la revendication 21 ou 22, dans lequel le moyen pour amplifier l'acide nucléique de VHB est un moyen d'amplification par réaction en chaîne par polymérase.

24. Kit selon l'une quelconque des revendications 21 à 23, qui comprend un oligonucléotide qui comprend une séquence choisie dans le groupe comprenant la SEQ ID NO: 8, la SEQ ID NO: 9, la SEQ ID NO: 10, la SEQ ID NO: 11, et leur séquence complémentaire, marqué de manière détectable et utile comme sonde.

25. Kit selon la revendication 24, dans lequel ledit oligonucléotide qui comprend une séquence choisie dans le groupe comprenant la SEQ ID NO: 8, la SEQ ID NO: 9, la SEQ ID NO: 10, la SEQ ID NO: 11, et leur séquence complémentaire, est la SEQ ID NO: 12, la SEQ ID NO: 13, la SEQ ID NO: 14 ou la SEQ ID NO: 15.


### SEQUENCE LISTING

<110> BIO-RAD Pasteur
<120> Olignonucleotides for the detection of hepatitis B virus
<130> BET 03P0671
<160> 15
<170> "PatentIn version 3.1
<210> 1
   <211> 3215
   <212> DNA
   <213> Hepatitis B virus
<400> 1
   ctccaccact ttccaccaaa ctcttcaaga tcccagagtc agggccctgt accttcctgc     60
   tggtggctcc agttcaggaa cagtgagccc tgctcagaat actgtctctg ccatatcgtc    120
   aatcttatcg aagactgggg accctgtgcc gaacatggag agcatcgcat caggactcct    180
   aggacccctg ctcgtgttac aggcggggtt tttcttgttg acaaaaatcc tcacaatacc    240
   acagagtcta gactcgtggt ggacttctct caattttcta gggggaacac ccgtgtgtct    300
   tggccaaaat tcgcagtccc aaatctccag tcactcacca acctgttgtc ctccaacttg    360
   tcctggttat cgctggatgt gtctgcggcg ttttatcatc ttcctctgca tcctgctgct    420
   atgcctcatc ttcttgttgg ttcttctgga ctatcaaggt atgttgcccg tttgtcctct    480
   aattccagga tcatcaacca ccagcacggg accatgcaag acttgcacag ctcctgctca    540
   aggaacctct atgtttccct catgttgctg tacaaaacct acggacggaa actgcacctg    600
   tattcccatc ccatcatctt gggctttcgc aaaataccta tgggagtggg cctcagtccg    660
   tttctcttgg ctcagtttac tagtgccatt tgttcagtgg ttcgtagggc tttcccccac    720
   tgtctggctt tcagttatat ggatgatgtg gttttggggg ccaagtctgt acaacatctt    780
   gagtcccttt ataccgctgt taccaatttt cttttgtctt tgggtataca tttaaaccct    840
   cacaaaacaa aaagatgggg atattccctt aacttcatgg gatatgtaat tgggagttgg    900
   ggcacattgc cacaggaaca tattgtacaa aaaatcaaaa cgtgttttag gaaacttcct    960
   gtaaacaggc ctattgattg gaaagtatgt caacgaattg tgggtctttt ggggtttgcc   1020
   gcccctttca cgcaatgtgg atatcctgct ttaatgcctt tatatgcatg tatacaagca   1080
   aaacaggctt ttactttctc gccaacttac aaggcctttc taagtaaaca gtatctgaac   1140
   ctttaccccg ttgctcggca acggcctggt ctgtgccaag tgtttgctga cgcaaccccc   1200
   actggttggg gcttggccat aggccatcag cgcatgcgtg gaacctttgt gtctcctctg   1260
   ccgatccata ctgcggaact cctagccgct tgttttgctc gcagcaggtc tggggcaaaa   1320
   ctcatcggga ctgacaattc tgtcgtgctc tcccgcaagt atacatcctt tccatggctg 1380
   ctaggctgtg ctgccaactg gatcctgcgc gggacgtcct ttgtttacgt cccgtcggcg 1440
   ctgaatcccg cggacgaccc ctcccggggc cgcttggggc tctaccgccc gcttctccgc 1500
   ctgttgtacc gaccgaccac ggggcgcacc tctctttacg cggactcccc gtctgtgcct 1560
   tctcatctgc cggaccgtgt gcacttcgct tcacctctgc acgtcgcatg gagaccaccg 1620
   tgaacgccca caggaacctg cccaaggtct tgcataagag aactcttgga ctttcagcaa 1680
   tgtcaacgac cgaccttgag gcatacttca aagactgtgt gtttactgag tgggaggagt 1740
   tgggggagga ggttaggtta atgatctttg tactaggagg ctgtaggcat aaattggtgc 1800
   gttcaccagc accatgcaac tttttcacct ctgcctaatc atctcttgtt catgtcctac 1860
   tgttcaagcc tccaagctgt gccttgggtg gctttgggac atggacattg acccgtataa 1920
   agaatttgga gcttctgtgg agttactctc ttttttgcct tctgacttct ttcctgctgt 1980
   tcgagatctc ctcgacaccg cctctgctct gtatcgggag gccttagagt ctccggaaca 2040
   ttgttcacct caccatacgg caatcaggca agctattctg tgttggggtg agttgatgaa 2100
   tctagccacc tgggtgggaa gtaatttgga agatcaagca tccagggact tagtagtcag 2160
   ctatgtcaac gttaatatgg gcctaaaatt cagacaacta ttgtggtttc acatttcctg 2220
   tcttacgttt gggagacaaa ctgttcttga atatttggtg tcctttggag tgtggattcg 2280
   cactcctcct gcatatagac caccaaatgc ccctatctta tcaacacttc cggaaactac 2340
   tgttgttaga caaagaggca ggacccctag aagaagaact ccctcgcctc gcagacgaag 2400
   gtctcaatcg ccgcgtcgca gaagatctca atctcgggaa tctcaatgtt agtattcctt 2460
   ggacacataa ggtgggaaac tttactgggc tttattcttc tacggtacct tgctttaatc 2520
   ctaattggca aactccttct tttcctgaca ttcatttgca ggaggacatt gttgatagat 2580
   gtaagcaatt tgtggggccc cttacagtaa atgaaaacag gagactaaaa ttaattatgc 2640
   ctgctaggtt ttatcccaat gttactaaat atttgccctt agataaaggg atcaaaccgt 2700
   attatccaga gtatgtagtt aatcattact tccagacgcg acattattta cacactcttt 2760
   ggaaggcggg gatcttatat aaaagagagt ccacacgtag cgcctcattt tgcgggtcac 2820
   catattcttg ggaacaagat ctacagcatg ggaggttggt cttccaaacc tcgaaaaggc 2880
   atggggacaa atcttgctgt ccccaatccc ctgggattct tccccgatca tcagttggac 2940
   cctgcattca aagccaactc agacaatcca gattgggacc tcaacacgca caaggactac 3000
   tggccggacg catggaaggt gggagtggga gcattcgggc cagggttcac ccctccccat 3060
   gggggactgt tggggtggag ccctcaggct cagggcctac tcacaactgt gccagcagct 3120
   cctcctcctg cctccaccaa tcggcagtca ggaaggcagc ctactccctt atctccacct 3180
   ctaagagaca ctcatccaca ggccatgaag tggaa 3215
<210> 2
   <211> 18
   <212> DNA
   <213> Artificial : oligonucleotide
<400> 2
   gctgaatccc gcggacga 18
<210> 3
   <211> 21
   <212> DNA
   <213> Artificial : oligonucleotide
<400> 3
   gtgcagaggt gaagcgaagt g 21
<210> 4
   <211> 19
   <212> DNA
   <213> Artificial : oligonucleotide
<400> 4
   gttcacggtg gtcgccatg 19
<210> 5
   <211> 19
   <212> DNA
   <213> Artificial : oligonucleotide
<400> 5
   gttcacggtg gtctccatg 19
<210> 6
   <211> 21
   <212> DNA
   <213> Artificial : oligonucleotide
<400> 6
   cgttcacggt ggtcgccatg c 21
<210> 7
   <211> 21
   <212> DNA
   <213> Artificial oligonucleotide
<400> 7
   cgttcacggt ggtctccatg c 21
<210> 8
   <211> 22
   <212> DNA
   <213> Artificial : oligonucleotide
<400> 8
   ggagtccgcg taaagagagg tg 22
<210> 9
   <211> 22
   <212> DNA
   <213> Artificial : oligonucleotide
<400> 9
   ggagaccgcg taaagagagg tg 22
<210> 10
   <211> 22
   <212> DNA
   <213> Artificial : oligonucleotide
<400> 10
   ggagtctgcg taaagagagg tg 22
<210> 11
   <211> 22
   <212> DNA
   <213> Artificial : oligonucleotide
<400> 11
   ggagactgcg taaagagagg tg 22
<210> 12
   <211> 32
   <212> DNA
   <213> Artificial : oligonucleotide
<400> 12
   cggcaggagt ccgcgtaaag agaggtgtgc cg 32
<210> 13
   <211> 32
   <212> DNA
   <213> Artificial : oligonucleotide
<400> 13
   cggcaggaga ccgcgtaaag agaggtgtgc cg 32
<210> 14
   <211> 32
   <212> DNA
   <213> Artificial : oligonucleotide
<400> 14
   cggcaggagt ctgcgtaaag agaggtgtgc cg 32
<210> 15
   <211> 32
   <212> DNA
   <213> Artificial : oligonucleotide
<400> 15
   cggcaggaga ctgcgtaaag agaggtgtgc cg 32